Europäisches Patentamt

European Patent Office

Office européen des brevets

(11) Publication number: **0 304 970**
**A2**

## EUROPEAN PATENT APPLICATION

(21) Application number: **88201089.5**

(22) Date of filing: **27.03.84**

(51) Int. Cl.⁴: **A61B 5/00 , A61B 5/02**

(43) Date of publication of application:
**01.03.89 Bulletin 89/09**

(60) Publication number of the earlier application in accordance with Art.76 EPC: **0 157 962**

(84) Designated Contracting States:
**DE FR GB IT**

(71) Applicant: **HEMODYNAMICS TECHNOLOGY, INC.**
**2694 Lavery Court, No. 20A**
**Newbury Park California 91320(US)**

(72) Inventor: **Bitterly, Jack G.**
**4723 Vista De Oro Avenue**
**Woodland Hills California(US)**

(74) Representative: **Gold, Tibor Zoltán et al**
**T.Z.GOLD & COMPANY 9, Staple Inn**
**London WC1V 7QH(GB)**

(54) Heat exchanger apparatus for cooling an object.

(57) A heat exchanger (31) cools at a programmed rate a predetermined area of skin. The rate at which the area of skin changes in response to temperature change of the heat exchanger and the rate the skin temperature returns to normal are then measured and compared to standard rates over a number of cycles. Impaired peripheral blood flow is evident especially on subsequent cycles as vascular disease slows the rate of return to normal temperature of the skin. The heat exchanger has a cooling chamber (110) with one side of the chamber being a cooling surface (111) adjacent the skin. An injector (130) is connected to a source of fluid (75) having a boiling point at ambient pressure less than the temperature to which the skin is to be cooled. The injector injects fluid into the chamber and onto the side of the cooling surface opposite the skin to boil the fluid on the surface to cool the surface and the skin.

Fig. 3.

## Heat Exchanger Apparatus for Cooling an Object

This invention relates to heat exchanger apparatus for cooling an object; and finds particular but not exclusive use in a method and apparatus for monitoring and diagnosing peripheral vascular blood flow (PVD) described and claimed in European Patent Application No. 84302083.5 (Publication No. 0 157 962) from which the present application has been divided.

According to the present invention, there is provided a heat exchanger apparatus which is as claimed in claim 1. When used for diagnosing PVD, cooling is accomplished by mounting such heat exchanger on a cuff attached at or around an extremity. The heat exchanger has a unique system that injects a liquid into a cooling chamber. The liquid boils from skin temperature heat energy at atmospheric pressure and cools the surface of the chamber that contacts the skin, thus cooling the skin quickly to a preferably pre-set minimum temperature. Then the heat exchanger is turned off, and the skin temperature begins to rise back to its normal temperature. A second cycle is then initiated. Even in normal individuals, the time necessary to return the skin temperature to normal usually increases on subsequent cycles.

After a given number of cycles one views and compares a graph of the temperature/time chart to a 'normal' graph. In persons with PVD, the rate at which skin temperature drops and returns to normal will change, and each cycle will yield a greater change. One who is experienced can evaluate the graph, or the graph can be analyzed mathematically.

The heat exchanger is designed to cool to a precise temperature. Timing and rate of temperature of the heat exchanger are precisely controlled. The lag time from which start or stop commands are initiated is also minimized.

A preferred embodiment of the present invention also includes a hydraulic amplifier, which requires only minute amounts of electrical energy, for controlling low pressure liquid or gas, and the low pressure liquid or gas then controls the higher pressure liquid that is vaporized in the heat exchanger. This permits a portable and power-independent miniaturized system.

## BRIEF DESCRIPTION OF THE DRAWINGS

FIG. 1 is a side elevation of the cooling cuff, which contains the heat exchanger of the present invention, the cuff being shown on a patient's leg;

FIG. 2 is a perspective view of the cuff which shows the heat exchanger;

FIG. 3 is a sectional elevation of the heat exchanger that is attached to the patient's extremity;

FIG. 4 is a plan sectional view of the heat exchanger taken through plane IV-IV in FIG. 3;

FIG. 5 is a graph showing skin temperature changes over time for two patients being monitored by the present invention;

FIG. 6 is an exploded view in perspective of the portion of the apparatus of the present invention that controls the heat exchanger itself; and

FIG. 7 is a block diagram embodying the present invention.

## DETAILED DESCRIPTION OF THE PREFERRED EMBODIMENT

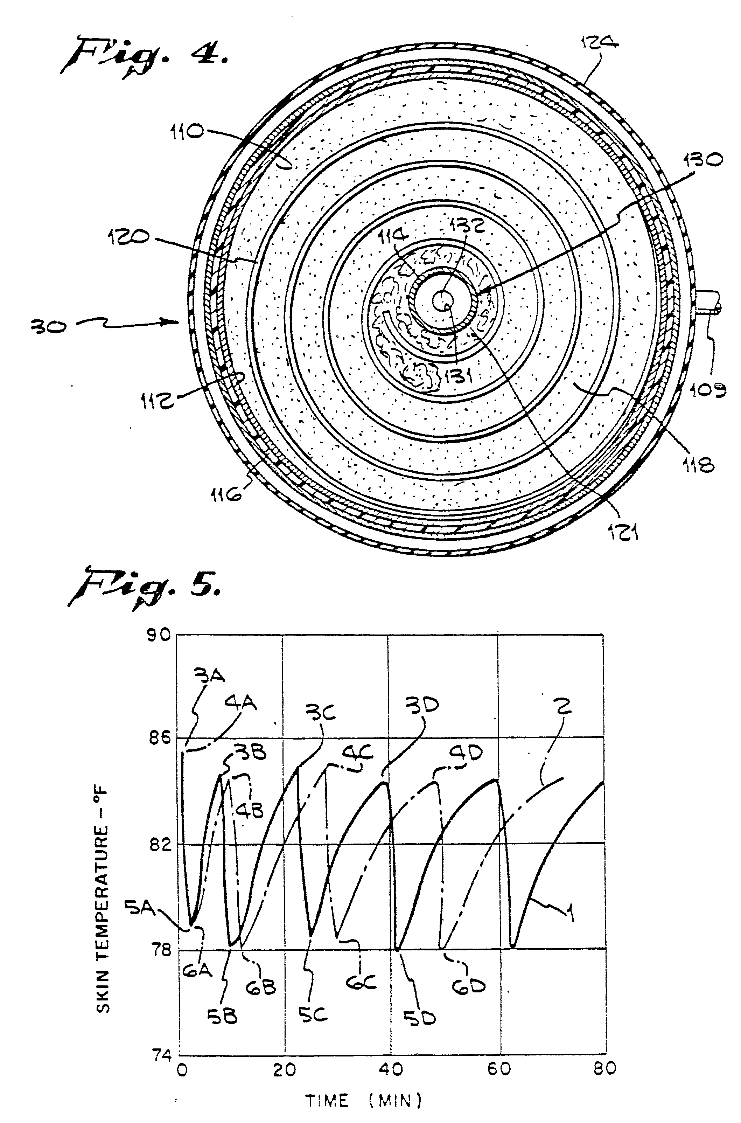

When a cold or warm object is applied to an area of the skin, the rate at which the skin cools or warms and at which the body returns that portion of skin to its normal temperature can be correlated to the efficiency of the vascular supply system. The present invention relies upon this principle when one cools the same area of skin after the skin temperature recovers to normal after a previous cooling, the second return to normal will take longer. (Cooling is used in the preferred embodiment for reasons set forth below.) As this process is repeated, the length of time for recovery will continue to increase. In FIG. 5 skin temperature is plotted against time. Curve 1 is representative of the temperature/time curve for an adult male suffering no PVD. Curve 2, superimposed in FIG. 5, is a hypothetical curve for the same size male suffering some PVD. The present invention offers a safe and inexpensive way of obtaining various curves. Because thermodynamics of the skin accurately and reproducibly reflect aspects of a person's vascular condition, by comparing curves 1 and 2, the person's vascular condition can be observed under controlled but somewhat variable conditions without introducing significant measuring artifacts.

Cooling rather than heating is used for a number of reasons. First, persons are accustomed to having their skin exposed to relatively cold surfaces down to approximately 10°C (50°F). The same temperature increase to the skin could be painful or at least uncomfortable. Second, the vapor phase-change heat exchanger of the present invention, a small lightweight and accurate device, cools very quickly and safely.

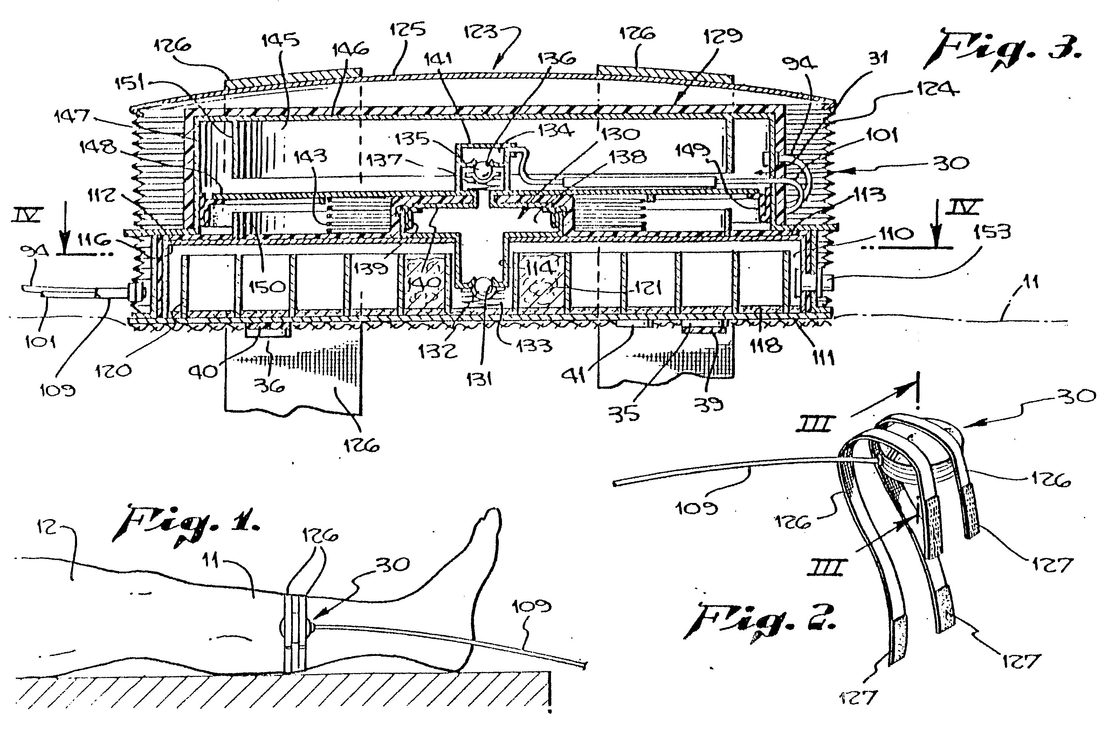

The cooling cuff 30 (FIGS. 1 and 2) is attached to the calf portion 11 of the patient's leg 12 by one or more straps 126 (two are shown) connected at their ends by hook and loop fasteners 127. The cuff can be made small enough to attach to a finger or toe.

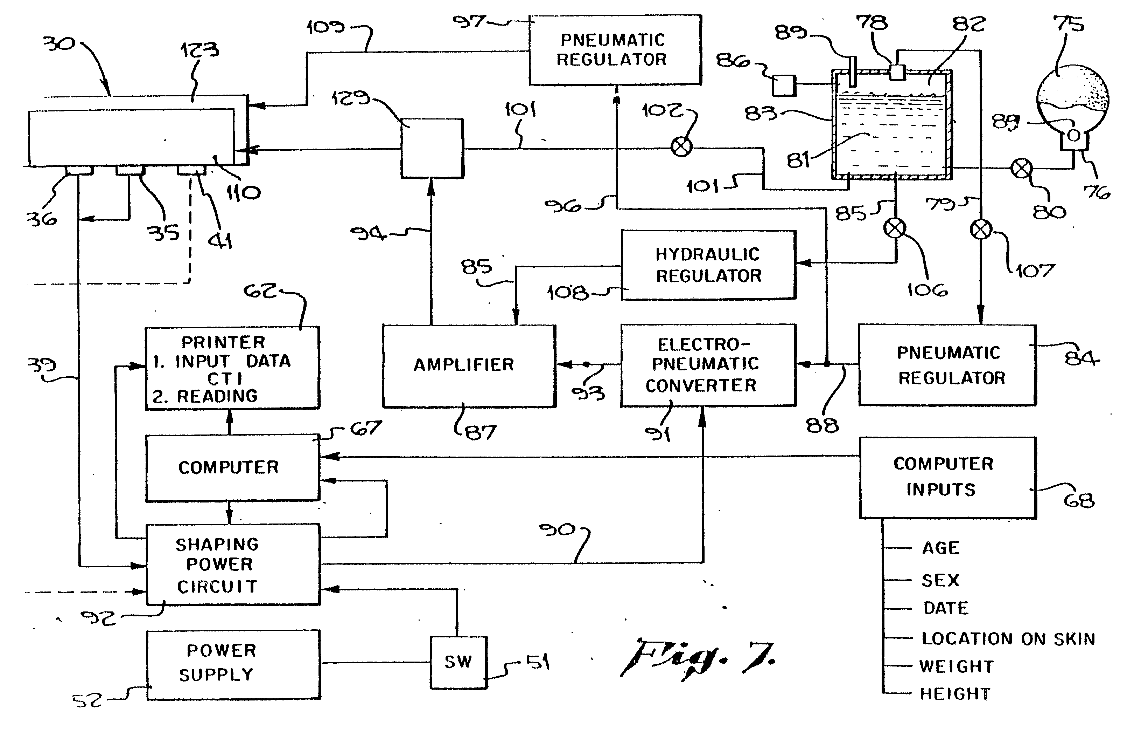

Although cooling could be accomplished in many ways, the present heat exchanger is described in FIGS. 3 and 4, and the schematic operation is shown in part in FIG. 7. The heat exchanger 31 has a cooling chamber 110, which is formed from a bottom heat exchanger surface 111, a cylindrical sidewall 112 and a top wall 113. Part of injector 130 extends downward through opening 114 in top wall 113. The sidewall 112 and top wall 113 are covered with insulation 116 to limit heat transfer from the outside through those walls into the chamber. Cooling surface 111 should be formed of a high heat transmitting material. Gold and silver offer the best heat transmission, but copper or aluminum may be acceptable alternatives especially if plated with a protective non-corroding layer of gold. Cooling surface 111 may be flat, or it may be curved to accommodate the particular appendage being monitored.

Injecting means, which is connected to a source of pressurized liquid having a boiling point at atmospheric pressure less than the temperature to which the skin is to be cooled, injects the liquid into chamber 110 against surface 111. The heat transmitted from the body through surface 111 heats the liquid to cause it to boil at atmospheric pressure. The phase change from liquid to gas extracts heat from surface 111 which in turn extracts heat from the skin thereby cooling the skin. Although the injecting means 130 is described in further detail below, liquid under pressure is injected through orifice 131 (FIG. 3), which is sealed by spring 133 biased check ball valve 132.

As the liquid is injected through orifice 131, it should contact surface 111. To facilitate distribution of the liquid radially outward along surface 111, wick 118 on surface 111 causes the liquid to flow along the entire surface. Wick 118 may be any wicking material such as fibers, porous stones or screens. A wick may also surround spring 133 to transport the liquid to surface 111 and wick 118. In case the heat exchanger is not oriented with cooling surface 111 horizontal, guide means, a spiral wall 120 (FIGS. 3 and 4) on surface 111, are provided for transporting the fluid from the injecting means to spaced apart locations on the surface. The fluid that is injected into the chamber cannot, therefore, immediately fall to one location on sidewall 112. A secondary wick 121 in the form of a fabric or other wicking material in the first winding or two of the spiral directs fluid to the primary wick 118 when the unit is not mounted horizontally. Thereafter, the primary wick 118 will draw fluid along the spiral 120 so that it is exposed to heat transfer surface 111. Spiral 120 may have holes or slots near wick 118, which are filled with the wicking material, so that fluid can be transported radially outward in addition to the spiral path.

The remaining elements of the injecting means are activated by hydraulic control, but the underlying control system upstream from the injecting means is described first.

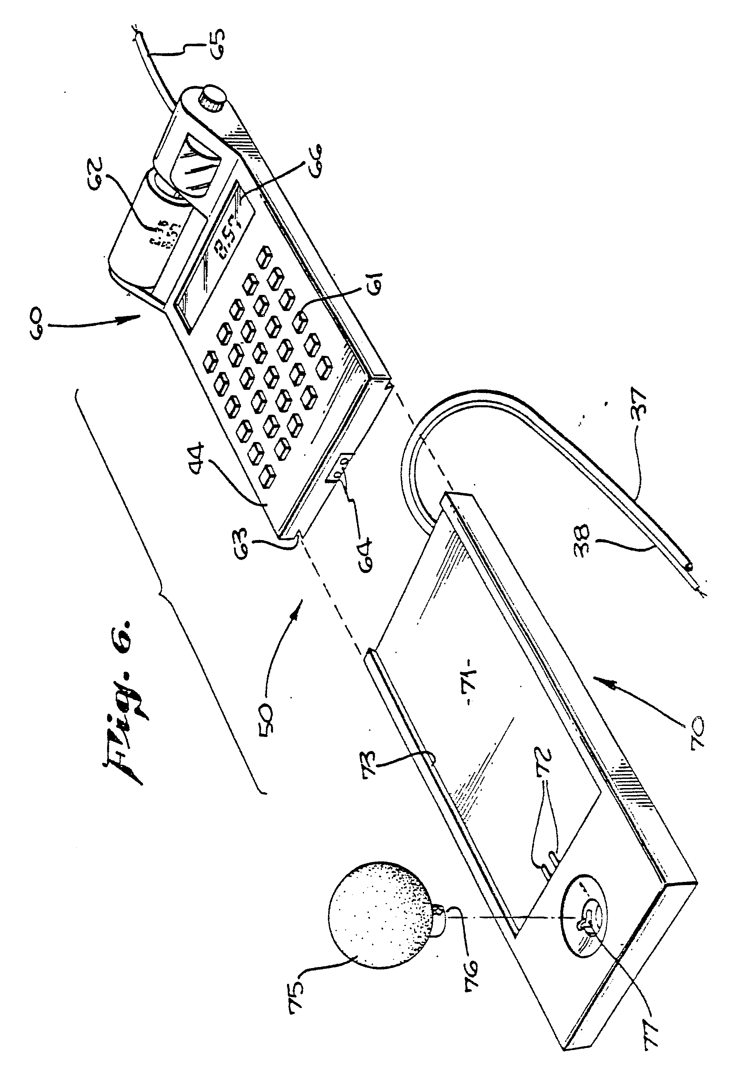

Energy to operate the various valves necessary for controlling cooling is obtained from the pressurized fluid in chamber 75 (FIGS. 6 and 7). Chamber 75 is filled with a propellant e.g. Freon, mixture under pressure so that it is in liquid form. Chamber 75 may be disposable and is approximately 3 - 5 cm in diameter. A male bayonet fitting 76 at the bottom of chamber 75 mates with a female receiving member 77 in base 70. These valves could operate together so that both open only if the male member 76 of chamber 75 is properly inserted in the female receiving member 77. A secondary opening in chamber 75 or valve 76 may be used for filling the chamber 75.

Only the liquid phase should flow out of chamber or tank 75. In that regard, a leveling sensor 86 (FIG. 7), which is known in the art, is permanently mounted on the base in any desired location (it is shown attached to tank 83 in FIG. 7) to give an audible or other warning or provide a signal to close valve 80 if base 70 is not level sufficiently while chamber 75 is mounted on the base 70. Alternatively, the control system could shut valves 102, 106 or 107 in any combination if base 70 is not relatively horizontal.

Liquid from tank 75 then flows past valve 80 to fill secondary or internal tank 83. A vent 89 may be provided to allow gas at the top of tank 83 to escape to atmosphere during filling. Escape of liquid from vent 89 indicates that tank 83 is filled. The bottom of vent 89 should extend downward slightly below gas outlet 78 to provide a reservoir 82 of gas. A number of lines with appropriate valves and a vent and gas outlet lead from tank 82. These could be provided in chamber 75, but that would complicate the chamber. As chambers 75 may be disposable, it would be undesirable to further complicate them. Therefore, the intermediate tank 83 can accomplish all of the functions of a more complicated tank 75. Each cycle therefore costs less. Other ways of utilizing both the gas and liquid and controlling them could also be provided.

The inside of tank 83 is shown in FIG. 7. Although it is shown rectangular, the shape can be modified. The fluid is divided into a liquid phase 81 and a gaseous or vapor phase 82. The fluid is a mixture of Freon fluorocarbons having particular vaporization characteristics and having safe breathing characteristics during venting and normal operation. The mixture is chosen such that the boiling point of the gas cools heat exchanger 31 (FIG. 3) to a chosen temperature at a chosen rate. The mixture boils below normal skin temperature 37° C but well above freezing 0° C so that frostbite does not occur.

One outlet 78 of tank 83 (FIG. 7) is located at the top of the tank so that only gas is discharged, and the gas passes through line 79 to valve 107 into pneumatic regulator 84. The miniature pneumatic regulator lowers the pressure to a predetermined regulated pressure. The pressure in line 79 may vary with temperature of the fluid, and it is desirable to use a regulated pressure for controlling. Hydraulic line 85, which is valved at 106, carries liquid, which is reduced in pressure by hydraulic regulator 108, to one input of amplifier 87. The low pressure gas from regulator 84 is directed through line 88 into electro-pneumatic converter 91, which is actuated by an electrical signal through line 90 from shaping power circuit 92. Circuit 92 converts the analog signal from the sensors, such as thermistors 35 and 36 from line 39, to a useable signal.

When a cooling cycle begins, circuit 92 signals electropneumatic converter 91 to permit gas to flow through line 93 into pneumatic amplifier 87. By using low pressure gas in the electro-pneumatic converter 91, only a small valve is needed. Thus, only a small amount of power in line 90 is needed to open the electro-pneumatic converter. Utilizing higher pressure gas or liquid would require more of a power drain. When electro-pneumatic converter 91 opens, gas flows through line 93 into an input in fluid amplifier 87. Fluid amplifiers are well known. Some are discussed in Tool Engineers Handbook, 2d at 12-15 et seq. (1959). One hydraulic amplifier is discussed in detail in describing Fig. 3. When electro-pneumatic converter 91 opens and gas flows through line 93, amplifier 87 injects hydraulic fluid from line 85 into line 94 at a higher pressure, if desired.

Cuff 30 (FIG. 3) includes a ballonet about the heat exchanger 31. Ballonet 123 comprises a bellows wall 124 and a somewhat flexible top wall 125. Cuff 30 is loosely attached to the skin using straps 126. A portion of the gas, bled from line 88, is directed through line 96 to a pneumatic regulator 97, which can be mounted in base 70 at or near cuff 30. Low pressure gas from pneumatic regulator 97 is then directed through line 109 into ballonet 123 (Fig. 7). By a predetermined regulation, the pressure in ballonet 123 applies a correct pressure of surface 111 to the skin. If the pressure is too great, the cuff will interfere with circulation and affect readings. Surface 111 and the sensors 35, 36 and 41 should be in contact with the skin. A ballonet gauge pressure of between 1.7 and 3.5 kPa is desirable.

Liquid 81 in tank 83 also flows into line 101 (FIG. 7) where it is controlled by value 102. Valves 102 and 106 could be combined with line 85 being connected to line 101 downstream of valve 102, or line 101 could be connected to line 85 downstream from valve 106. Relatively high pressure liquid enters injector system 129 (FIGS. 3 and 7) from line 101, and a lower pressure fluid enters through line 94.

Injector system 129 is located within cuff 30 (FIG. 3) but it could be outside the cuff (see FIG. 7). It is desirable if all lines 94, 101, and 109 entered the cuff through one inlet. Flexible, small diameter tubing with three or more attached tubes is available, and even the electrical leads from sensors 35, 36 and 41 may be attached to the tubes in spaces between them. Preferably, they would all enter the cuff through bellows wall 124 near the skin surface so as not to apply a torque to the cuff and the heat exchanger (FIGS. 2 and 3).

High pressure liquid in line 101 (FIG. 3) enters upper chamber 134 where it flows through orifice 136 into main chamber or cell 138 of injector 130. Check valve 135 is urged against orifice 136 by spring 137. Chamber 138 is formed in part of piston wall 139 which is flexible to allow top wall 140 and upper chamber wall 141 to more up and down in response to pressurization of chambers 138 and 145. The spring constants of springs 137 and 133 for check valves 135 and 132 are chosen such that normal maximum pressure in line 101 is sufficient to overcome spring 137 and allow liquid to flow past check valve 135 into main chamber 138, but the pressure is insufficient to overcome the force of spring 133 acting on check valve 132. Therefore, the high pressure liquid is contained within chamber 138.

The lower pressure liquid through line 94 is fed into control chamber or cell 145 (FIG. 3), which consists of top wall 146 and sidewall 147. Bottom wall 148 is mounted to wall 147 by flexible member 149 which permits bottom wall 148 to move up and down between lower ring stop 150 and upper ring stop 151. Upper chamber 134 and top wall 140 of injector 130 are connected to bottom wall 148. Spring 143 and pressure inside of chamber 138 urges bottom wall 148 upward against stop 151.

When fluid is injected through line 94, the pressure exerts a downward force on bottom wall 148. Because the area of bottom was 148 is very much greater than the area of wall 140 of the main chamber 138, lower pressure in chamber 145 is amplified to force top wall 140 and top chamber 141 downward against the pressure in chamber 138 acting on wall 140. Bottom wall 148 moves down until it contacts bottom ring stop 150. This action greatly pressurizes the inside of main chamber 138 which overcomes the spring force of spring 133 and allows the liquid to be injected through orifice 131 into chamber 110. The check valve 135 prevents the higher pressure liquid from flowing back into line 101.

Thereafter, the pressure in line 94 is vented, and new liquid flows through line 101 past orifice 136 into main chamber 138 which creates an upward force in conjunction with spring 143 on wall 148 to allow main chamber 138 to fill again.

The injection system 129 injects a precise amount of the liquid into cooling chamber 110. Minimum power is utilized, and the pressurized working fluids do the work. Neither large electrical power nor high voltage is necessary, and the system is very compact. Also by utilizing a liquid working fluid close the heat exchanger, time lags are substantially reduced or eliminated, and the computing system does not have to compensate for such lags. Changes in outside temperature also have less affect.

Only liquid, not gas, is injected to heat exchanger surface 111. No latent heat of vaporization occurs except where it is wanted on surface 111. The vaporized liquid is vented as gas out of vent 153. (FIGS. 3 and 4).

The initiation of the cooling cycle is shown as points 3A, 3B, 3C and 3D on curve 1 and for curve 2, cooling starts at points 4A, 4B, 4C, and 4D (FIG. 5). Although curves 1 and 2 assume a long test with approximately 3.3°C(6°F) temperature changes, the control system permits smaller changes in temperature to decrease test time. The control system stops cooling when the temperature of the skin or heat exchanger reaches a predetermined point or after a predetermined interval. In one test, the designed heat exchanger cooled at approximately 1.7°C (3.1°F) per second so that a temperature drop of approximately 17°C (30°F) occurs in ten seconds. The shutoff points along curve 1 are indicated as points 5A, 5B, 5C, and 5D and the shutoff points for curve 2 are indicated as 6A, 6B, 6C, and 6D.

Once the heat exchanger starts cooling, the body works to maintain and return the skin temperature to its normal state. The rate at which the skin temperature drops and the rate at which it returns to normal is correlated to the efficiency of the peripheral vascular system. Skin temperature of one with an inefficient vascular system will drop more quickly than that of a normal person. The efficient system may not drop as far, and it will return to normal more quickly.

In one test, after the skin reaches its normal or slightly below normal temperature at points 3, 4, etc.,- (FIG. 5), the cooling is initiated again. The body returns the area to normal more slowly on later cycles.

The healthy person's skin temperature takes longer to return to normal on the second cycle (point 5A to point 3C) than it does on the first cycle (point 5A to point 3B). The person with some disease, however, not only takes longer to return to a normal skin temperature after the first cycle (from 6A to 4B), but the rate of return to normal temperature slows to an even greater degree during subsequent cycles.

The device plots temperature curves, which trained persons can then read and diagnose conditions. One can also analyze the data mathematically to derive a numerical indicator. Because of the change in rates of return of skin temperature to normal between different subjects, the area under those curves can be determined by integration using computer analysis to generate an index, which is called the cardio-thermal index or CTI. CTI may be defined, for example, as the summation of the integrals of the temperature recovery portion of the curve over a given number of cycles divided by the summation of the integrals of the cooling portion. The result is non-dimensional, and the computer can express the result of a digital number of percentage of normal. The equation is thus:

$$CTI = \frac{\left\{ \Sigma_c \int_{n-1}^{n} T(t)dt \right\}_c}{\left\{ \Sigma_\omega \int_{n-1}^{n} T(t)dt \right\}_\omega}$$

where dt is the time differential; T(t) = skin temperature variation as a function of time, t, and temperature, T. $\Sigma_L$ is a summation of all forced cooling integrals to be considered from the first curve through a given number of odd numbered curves. Referring to FIG. 5, the odd cycles are the cooling ones and include the first cycle (3A to 5A), the third cycle (3B to 5B), etc. Each curve is integrated. $\Sigma_\omega$ is a summation of all warming integrals being considered from the second curve through a given even number of curves, which is one larger than the given number during cooling. In Fig. 5, the even cycles are warming cycles and include the second cycle (5A to 3B), the fourth cycle (5B to 3C), etc. Each of those curves is integrated. The resulting CTI is very sensitive to blood flow in the capillary bed.

The temperature cycling is believed to cause the construction and dilation of the arterioles to respond less efficiently during each succeeding cycle (similar to muscle fatigue after strenuous work). The effect is more pronounced in persons with PVD. In short, there are two effects - (1) a single cycle effect that primarily shows blood flow rate in the capillary bed; and (2) a frequency damping effect, indicating the tone or normalcy of the arterioles. Both effects can be examined, separated and manipulated by desired mathematical analysis and displayed by standard computer technology.

The testing and data interpretation of the present invention can be done in many other ways, for example:

(a) Applying cooling at a fixed rate for a specific time. This will yield a fixed rate of heat removal from the skin in the covered area for the specified time. The index from such a test could be correlated on the basis of either an average time/temperature slope, the maximum cooling rate, or an integral of the time temperature curve over some specified time or temperature range;

(b) Maintaining the heat exchanger temperature for a specified period of time. Constant patch temperature cooling could be correlated in the same manner as constant cooling rate data;

(c) Applying cooling in a cyclical manner to generate a skin temperature excursion over a specified range. Data generated through cyclical heat removal may be correlated by plotting temperature recovery between cooling cycles versus the number of cooling cycles then by integrating this curve over the number of cycles generated; and

(d) Applying cooling cyclically at a fixed cooling rate but varying the on/off cycles over different frequencies.

In the control and monitoring system of the present invention (FIGS. 6 and 7), monitor 50 (FIG. 6) has two main parts, a console 60 and a base 70. Console 60 includes a keyboard 61 that is used to program the control system. The keyboard may control the on/off function, or a separate switch 51 (FIG. 7) may control the electrical battery power supply 52. Keyboard 61 also inputs at 68 in FIG. 7 different variables such as height, weight, age, sex and location on the skin that the cuff 30 is placed. The keyboard 61 could also be used to enter the patient and physician's name, the date and time of the test and other related information so that the printer 61 (FIGS. 6 and 7), which is a standard calculator printer, prints this data for record keeping purposes. The keyboard 61 is tied into computer 67 (FIG. 7) that monitors and controls the controller. The computer 67 includes a pre-programmed ROM for the basic system controls and for the computation mathematics.

The console 60 mates with base 70 in the dovetailing grooves 63 and 73 on console 60 and base 70, respectively, which holds the two units together. A lock may be provided to prevent separation of the base 70 from the console 60. When grooves 73 and 63 engage each other and console 60 is pushed entirely within receiving opening 72 on the base 70, male electrical connector 72 engages corresponding female electrical members 64 on the console 60. Sensed data from the temperature sensors in the cuff 30, transmitted through wires in cable 38 (FIGS. 6 and 7) to console 70 is transmitted through electrical connectors 72 and 64 (FIG. 6) to console 60.

In addition to the printer 62 for printing an alphanumerical CTI, the console could be connected through output line 65 (FIG. 6) to a graphic printer where the analog results of the test are printed and/or to a remote monitoring station in which either analog or digital results could be displayed, for example, at a hospital nursing station. The console 60 may also have a digital display 66 for instantaneously monitoring the CTI.

It is advantageous to minimize the electrical power used especially the power to cuff 30. Utilizing a small voltage for a temperature sensor is important for safety reasons. The small batteries that could be used to power the computer in console 60 pose no danger to the patient. Therefore, most of the cooling control is done hydraulically or pneumatically as shown above. The power requirements for the small computer housed within the console 60 are small enough for small battery power. The temperature sensors on the cuff 30 are thermistors 35 and 36 (FIG. 3), which respond to temperature changes by changes in resistance. A small voltage across the thermistors 35 or 36 is sufficient to read the temperature change. A platinum resistance thermistor (PRT) is desirable because of its linearity, but a computer can compensate for nonlinearity. A sensor 41 for measuring heat flux could also be provided for additional data. Because of insulation 39 between sensor 35 and the skin, sensor 35 reads the temperature of surface 111; sensor 36 reads skin temperature because of insulation 40 between the sensor and the surface 111.

## Claims

1. A heat exchanger apparatus (3) for cooling an object comprising:

a cooling chamber (110), one side of the chamber having a contact wall (111) for contacting the object to be cooled; characterised in that

injecting means (130) are connectable to a source of fluid (75), the fluid having a boiling point at ambient pressure less than the temperature to which the object is to be cooled, the injecting means being effective to inject fluid into the chamber (110) in a controllable manner and onto the said contact wall (11) to boil the fluid on the said wall (111) to cool it and the object.

2. A heat exchanger according to claim 1, characterised in that wick means (121) on the said wall (111) is effective to distribute the fluid along the said contact wall and there is a spiral wall (120) upstanding on the said contact wall (111) for guiding the fluid from the injecting means to spaced apart locations on the said contact wall (111).

3. Apparatus according to claim 1 or claim 2, characterised in that the source of fluid (75) contains liquid (81) and gas (82), a first set of lines (101) extending between the source of fluid (75) to a first chamber (138) in the heat exchanger (31) for carrying the liquid under pressure into the first chamber, a second line (75, 94) extending between the source of fluid (75) and a second chamber (145) of the heat exchanger (31) for carrying liquid to the second chamber in the heat exchanger, a movable wall (148) between the first and second chambers (138, 145) to transmit pressure forces from the liquid in the second chamber (145) to the liquid in the first chamber (138) to force the liquid in the first chamber (138) against the contact wall (111).

4. Apparatus according to any preceding claim, characterised in that the controlling means (92; 129) is effective to repeat in at least two distinct and successive cycles the injection of said fluid.

5. Apparatus according to claim 4, characterised in that said controlling means includes an electric circuit (92) connected to an electro-pneumatic converter (91) to permit said fluid in its gaseous phase to flow through said first set of lines (93, 101).

6. Apparatus according to any preceding claim, characterised in that dividing means (83) are provided for dividing fluid flow from the source (75) of fluid into two, low pressure and high pressure, fluid flow paths (85, 101) respectively,

first pressure reduction means (108) in the high pressure flow path for reducing the pressure in the flow path to a lower pressure, a first chamber (145) connected to the first pressure reduction means (108) for receiving the lower pressure fluid flow and having a first wall (148) movable in response to pressurized fluid from the pressure reduction means (108),

a second chamber (138) connected to the dividing means (83) for receiving high pressure fluid flow and having a second wall (140) having an area less than the area of the first wall (148) and being movable in response to movement of the first wall (148) to change the volume of the second chamber (138),

the second chamber (138) having an opening (131) to the chamber through which fluid can flow, check valve means (132) in the second chamber (138) across the opening (131) and being normally closed for blocking fluid flow through the opening (131) when the fluid in the second chamber (138) is at its normal, higher pressure, the check valve means (132) opening when movement of the second wall (140) decreases the volume of the second chamber (138) to inject fluid through the opening (131) into the chamber (138).

7. Apparatus according to claim 6, characterised in that there are first stop means (151) on one side of the first wall (148) for limiting travel of the first wall (148) in a direction to decrease the volume of the first chamber (145) to limit the maximum volume of the second chamber (138), second stop means (150) on the other side of the first wall (148) to limit travel of the first wall (148) in an opposite direction to limit the minimum volume of the second chamber (138) for precise volume change to control the amount of fluid injected into the chamber (138).

8. Apparatus according to claim 7, characterised in that higher pressure fluid from the source (75) of fluid flows into the second chamber (138) via an inlet (136) and a further check valve means (135) at the inlet means is effective to permit one-way flow of fluid through the inlet (136).

9. Apparatus according to any of claims 6 to 8, characterised in that a ballonet (123) is mounted about the heat exchanger, ballonet opening means through a portion of the ballonet (123) for exposing the said contact wall (111) therethrough, attaching means (126) on the ballonet for loosely attaching the ballonet (123) to the object, filling means (109) attached to the ballonet (123) for filing the ballonet with low pressure gas to urge the ballonet and the said contact wall (111) against the object at a controlled pressure.

Fig. 3.

Fig. 1.

Fig. 2.

EP 0 304 970 A2

*Fig. 4.*

*Fig. 5.*

Fig. 6.

EP 0 304 970 A2

Fig. 7.

EP 0 304 970 A2